# EUROPEAN PATENT APPLICATION

(11) **EP 4 154 869 A1**
(43) Date of publication of application: **29.03.2023**
(21) Application number: 21809050.4
(22) Date of filing: 24.05.2021
(51) Int. Cl.: A61K 9/08, A61K 47/26, A61K 47/18, A61K 47/12, A61K 47/22, A61K 47/20, A61K 47/64, A61K 38/00, A61K 38/26, A61P 3/04

(54) **LIQUID FORMULATION OF LONG-ACTING CONJUGATE OF GLP-2**

(30) Priority: 22.05.2020 KR 20200061878
(71) Applicant: Hanmi Pharm. Co., Ltd., Hwaseong-si, Gyeonggi-do 18536 (KR)
(72) Inventor: HONG, Sung Hee, Hwaseong-si, Gyeonggi-do 18469 (KR); KIM, Sang Yun, Hwaseong-si, Gyeonggi-do 18469 (KR); KIM, Min Young, Hwaseong-si, Gyeonggi-do 18469 (KR); BAE, Sung Min, Hwaseong-si, Gyeonggi-do 18469 (KR)
(74) Representative: Mewburn Ellis LLP
(86) International application number: PCT/KR2021/006455
(87) International publication number: WO 2021/235913

(57) **Abstract**

The present invention relates to a liquid formulation of a long-acting conjugate of glucagon-like peptide-2 (GLP-2) and a method for preparing the same.

## Description

### [Technical Field]

The present invention relates to a liquid formulation of a long-acting conjugate of glucagon-like peptide-2 (GLP-2) and a method for preparing the same.

### [Background Art]

Glucagon-like peptide-2 (GLP-2) is a peptide composed of 33 amino acids, which is produced by L cells in the small intestine in response to ingested nutrients, and has shown a high potential as a therapeutic agent for intestinal diseases, intestinal injuries and gastric diseases.

However, GLP-2 still has limitations in being developed into a commercial drug. Peptides such as GLP-2 can be easily denatured due to low stability, lose activity due to degradation by protease in the body, and are easily removed through the kidneys due to their relatively small size. Therefore, in order to maintain the blood concentrations and titers of drugs containing the peptide as a pharmacological ingredient, there is a need to administer the peptide drug more frequently. However, most peptide drugs are administered mostly in the form of injections, and frequent injections are required to maintain the blood concentration of the physiologically active peptide, which causes severe pain in patients. In particular, GLP-2 has extremely short *in vivo* half-life (7 minutes or shorter) due to the loss in the titer of GLP-2 by dipeptidyl peptidase-IV (DPP IV), which cleaves between the amino acids at position 2 (Ala) and position 3 (Asp) of GLP-2 (Bolette H. et al., The Journal of Clinical Endocrinology & Metabolism. 85(8):2884-2888, 2000). Accordingly, in order to increase the physiologically active half-life of GLP-2, the present inventors developed a long-acting conjugate of GLP-2 (Korean Laid-open Patent Publication No. 10-2010-0104382).

However, there is still a need to develop a stable liquid formulation that can store the long-acting conjugate of GLP-2 for a long time. Specifically, to supply drugs containing the long-acting conjugate of GLP-2 to drug products, it is necessary to maintain the pharmaceutical efficacy thereof *in vivo* while restraining physicochemical changes such as degeneration, aggregation, adsorption, or hydrolysis induced by light, heat, or impurities in additives during storage and transportation. Although the temperature, pH, and additives of the liquid formulation affect the rate of production of degradation products, compositions that stabilize all proteins so that they can be applied to clinical practice are not yet known, and in many cases, a liquid composition that exhibits a stabilizing effect in a specific protein may be not applied to stabilization of other proteins. It is known in the art that in order to maximize the stability of the target protein in the liquid phase, the selection of factors and additives capable of minimizing the production rate of specific degradation products of a protein and a combination thereof need to be extensively preceded.

In particular, in the case of a long-acting conjugate of GLP-2 with increased durability and stability *in vivo,* since GLP-2, a physiologically active peptide, and an immunoglobulin Fc fragment are linked, the molecular weight and volume are significantly increased than that of GLP-2, and thus, a special composition is required for protein stabilization. Additionally, since GLP-2, a physiologically active peptide, and an immunoglobulin Fc fragment are peptides or proteins with different physicochemical properties, the physiologically active peptide GLP-2 and the immunoglobulin Fc fragment must be stabilized simultaneously. However, different peptides or proteins may be progressively inactivated at different rates and under different conditions during storage due to their physicochemical differences, and when stabilizers suitable for each peptide or protein are used in combination, an adverse effect different from those intended may arise due to mutual competition and side effects. Further, during storage, the nature or concentration of the stored protein may change, leading to different effects. Therefore, in the case of a long-acting conjugate of GLP-2, it is difficult to find a composition of a stabilizer that simultaneously stabilizes the physiologically active peptide GLP-2 and the immunoglobulin Fc fragment.

### [Disclosure]

### [Technical Problem]

It is one object of the present invention to provide a liquid formulation of a long-acting conjugate of glucagon-like peptide-2 (GLP-2).

It is another object of the present invention to provide a method for preparing the liquid formulation.

### [Technical Solution]

One aspect of the present invention provides a liquid formulation of a long-acting conjugate of glucagon-like peptide-2 (GLP-2).

In one embodiment, the present invention relates to a liquid formulation of a long-acting conjugate of GLP-2, including:
a long-acting conjugate of GLP-2, in which GLP-2 and an immunoglobulin Fc fragment are linked to each other, in a pharmacologically effective amount, and
a stabilizer including a sugar alcohol, a saccharide, or a combination thereof; and a buffering agent.

In the liquid formulation according to any one of the preceding embodiments, the long-acting conjugate of GLP-2 is characterized in that it is represented by Chemical Formula 1 below:

[Chemical Formula 1] X-Lₐ-F

wherein,
X is native GLP-2 or a GLP-2 derivative;
L is a linker containing an ethylene glycol repeating unit;
a is 0 or a natural number, with the proviso that when a is 2 or more, each L is independent of each other;
F is an immunoglobulin Fc fragment; and
   - represents a covalent bond:

In the liquid formulation according to any one of the preceding embodiments, the liquid formulation is characterized in that it includes
18 nmol/mL to 18,630 nmol/mL of a long-acting conjugate of GLP-2 represented by Chemical Formula 1 above, and
a stabilizer including 1% (w/v) to 20% (w/v) of a sugar alcohol, a saccharide, or a combination thereof; and a buffer material in an amount for maintaining the pH in the range of 4.5 to 6.5:

In the liquid formulation according to any one of the preceding embodiments, the GLP-2 derivative is characterized in that it has a variation selected from the group consisting of substitution, addition, deletion, modification, and a combination thereof in one or more amino acids in the native GLP-2 sequence.

In the liquid formulation according to any one of the preceding embodiments, the X is characterized in that it includes an amino acid sequence represented by General Formula 1 below:

[General Formula 1] X₁X₂DGSFSDEMNTILDNLAARDFINWLIQTX₃₀ITDX₃₄ (SEQ ID NO: 9 9)

wherein,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent, or lysine, arginine, glutamine, histidine, 6-azidolysine or cysteine.

In the liquid formulation according to any one of the preceding embodiments, the X is characterized in that it is:
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine.

In the liquid formulation according to any one of the preceding embodiments, the X is characterized in that it includes an amino acid sequence represented by General Formula 2 below:

[General Formula 2] X₁X₂DGSFSDEMNTILDNLAARDFINWLIQTX₃₀ITDX₃₄ (SEQ ID NO: 10)

wherein,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or Aib (2-aminoisobutyric acid);
X₃₀ is lysine or arginine; and
X₃₄ is any one or more amino acids or any one or more amino acids in which a variation has occurred.

In the liquid formulation according to any one of the preceding embodiments, the GLP-2 derivative is characterized in that it is an amino acid sequence selected from the group consisting of SEQ ID NOS: 2 to 8.

In the liquid formulation according to any one of the preceding embodiments, the immunoglobulin Fc fragment is characterized in that it is an IgG4 Fc region.

In the liquid formulation according to any one of the preceding embodiments, the F is characterized in that it is a dimer consisting of two polypeptide chains, and one end of the L is linked to only one of the two polypeptide chains.

In the liquid formulation according to any one of the preceding embodiments, the L is characterized in that it is polyethylene glycol.

In the liquid formulation according to any one of the preceding embodiments, the formula weight of the ethylene glycol repeating unit moiety in the L is characterized in that it is in the range of 1 kDa to 100 kDa.

In the liquid formulation according to any one of the preceding embodiments, the immunoglobulin Fc fragment is characterized in that it includes the amino acid sequence of SEQ ID NO: 32.

In the liquid formulation according to any one of the preceding embodiments, the stabilizer is characterized in that it further includes one or more components selected from the group consisting of a nonionic surfactant and an amino acid.

In the liquid formulation according to any one of the preceding embodiments, the stabilizer is characterized in that it includes a sugar alcohol, a saccharide, or a combination thereof; a buffering agent; a nonionic surfactant; and an amino acid.

In the liquid formulation according to any one of the preceding embodiments, the liquid formulation may or may not include an isotonic agent.

In the liquid formulation according to any one of the preceding embodiments, the buffering agent is characterized in that it is selected from the group consisting of citric acid and a salt thereof, acetic acid and a salt thereof, histidine and a salt thereof, phosphoric acid and a salt thereof, and a combination thereof.

In the liquid formulation according to any one of the preceding embodiments, the pH of the liquid formulation is characterized in that it is in the range of 4.8 to 6.0.

In the liquid formulation according to any one of the preceding embodiments, the sugar alcohol is characterized in that it is one or more selected from the group consisting of mannitol and sorbitol.

In the liquid formulation according to any one of the preceding embodiments, the saccharide is characterized in that it is glucose, fructose, galactose, lactose, maltose, sucrose, or a combination thereof.

In the liquid formulation according to any one of the preceding embodiments, the nonionic surfactant is characterized in that it is poloxamer, polysorbate, or a combination thereof.

In the liquid formulation according to any one of the preceding embodiments, the nonionic surfactant is characterized in that it is selected from the group consisting of poloxamer 188, polysorbate 20, polysorbate 40, polysorbate 60, polysorbate 80, and a combination thereof.

In the liquid formulation according to any one of the preceding embodiments, the nonionic surfactant is characterized in that it is present in a concentration of 0.001% (w/v) to 0.2% (w/v) in the liquid formulation.

In the liquid formulation according to any one of the preceding embodiments, the amino acid is characterized in that it is selected from the group consisting of methionine, arginine, histidine, glycine, cysteine, and a combination thereof.

In the liquid formulation according to any one of the preceding embodiments, the amino acid is characterized in that it is present in a concentration of 0.01 mg/mL to 1 mg/mL in the liquid formulation.

In the liquid formulation according to any one of the preceding embodiments, the liquid formulation is characterized in that it includes:
18 nmol/mL to 18,630 nmol/mL of a long-acting conjugate of GLP-2;
5 mM to 100 mM of a buffering agent selected from the group consisting of citric acid and a salt thereof, acetic acid and a salt thereof, histidine and a salt thereof, phosphoric acid and a salt thereof, and a combination thereof;
1% (w/v) to 20% (w/v) of glucose, fructose, galactose, lactose, maltose, sucrose, mannitol, sorbitol, or a combination thereof;
0.001% (w/v) to 0.2% (w/v) of a nonionic surfactant selected from the group consisting of poloxamer, polysorbate, and a combination thereof; and
0.01 mg/mL to 1 mg/mL of an amino acid selected from the group consisting of methionine, arginine, histidine, glycine, cysteine, and a combination thereof, and
wherein the pH of the liquid formulation is 4.5 to 6.5.

In the liquid formulation according to any one of the preceding embodiments, the liquid formulation is characterized in that it includes:
18 nmol/mL to 18,630 nmol/mL of a long-acting conjugate of GLP-2;
5 mM to 100 mM of a buffering agent;
1% (w/v) to 20% (w/v) of a sugar alcohol, a saccharide, or a combination thereof;
0.001% (w/v) to 0.2% (w/v) of a nonionic surfactant; and
0.01 mg/mL to 1 mg/mL of an amino acid, and
wherein the pH of the liquid formulation is 4.5 to 6.5.

In the liquid formulation according to any one of the preceding embodiments, the liquid formulation is characterized in that it includes:
186 nmol/mL to 2,981 nmol/mL of a long-acting conjugate of GLP-2;
10 mM to 40 mM of a buffering agent of acetic acid and a salt thereof, or citric acid and a salt thereof;
2% (w/v) to 15% (w/v) of sucrose, mannitol, sorbitol, or a combination thereof;
0.002% (w/v) to 0.05% (w/v) of polysorbate; and
0.02 mg/mL to 0.5 mg/mL of methionine, and
wherein the pH of the liquid formulation is 4.8 to 6.0.

In the liquid formulation according to any one of the preceding embodiments, the liquid formulation is characterized in that it further includes polyhydric alcohol.

Another aspect of the present invention provides a method for preparing the liquid formulation.

In one embodiment, the present invention relates to a method for preparing the liquid formulation of any one of the preceding embodiments, including: mixing (i) the long-acting conjugate of GLP-2, in which GLP-2 and the immunoglobulin Fc fragment are linked to each other; with (ii) a stabilizer including a sugar alcohol, a saccharide, or a combination thereof; and a buffering agent.

### [Advantageous Effects]

The liquid formulation according to the present invention has the economic advantage by providing storage stability to the conjugate of the present invention having a large molecular weight through a simple formulation.

### [Brief Description of Drawings]

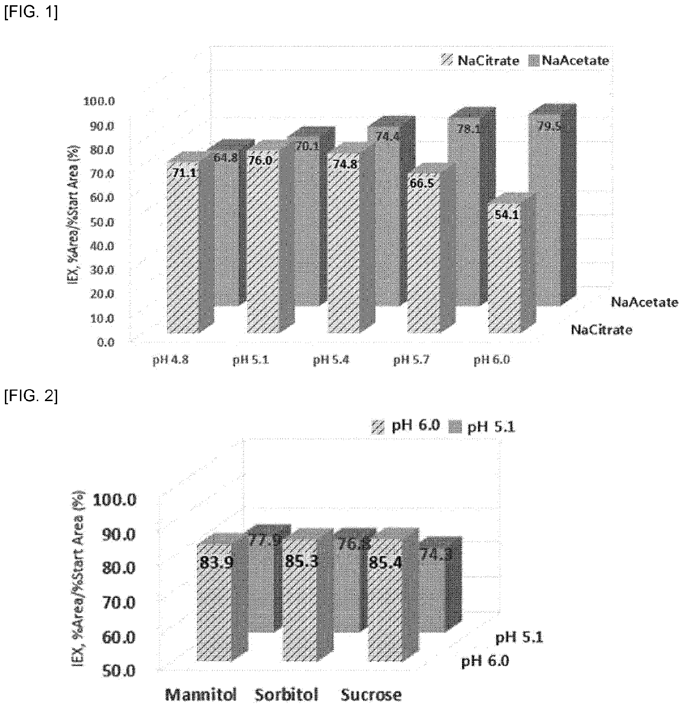

FIG. 1 is the result of confirming the stability of the long-acting conjugate of the GLP-2 derivative according to the types of buffering agents and the pH (IEX (%): IE-HPLC (%) in Table 3, residual rate of the long-acting conjugate of the GLP-2 derivative).
FIG. 2 is the result of confirming the stability of the long-acting conjugate of the GLP-2 derivative according to the types of stabilizers and the pH (IEX (%): IE-HPLC (%) in Table 5, residual rate of the long-acting conjugate of the GLP-2 derivative).

### [Detailed Description of the Invention]

Hereinafter, the present invention will be described in more detail.

Meanwhile, each of the explanations and exemplary embodiments disclosed herein can be applied to each other explanation and exemplary embodiment. That is, all of the combinations of various factors disclosed herein belong to the scope of the present invention. Moreover, the scope of the present invention should not be limited by the specific disclosure provided hereinbelow.

Additionally, those of ordinary skill in the art may be able to recognize or confirm, using only conventional experimentation, many equivalents to the particular aspects of the invention described herein. Furthermore, it is also intended that these equivalents be included in the present invention.

Throughout the entire specification of the present invention, not only the conventional one-letter and three-letter codes for naturally occurring amino acids, but also those three-letter codes generally allowed for other amino acids, such as α-aminoisobutyric acid (Aib), 6-azidolysine (_{AZ}K), *etc.* are used. Additionally, the amino acids mentioned herein are abbreviated according to the nomenclature rules of IUPAC-IUB as follows:

| | |
|---|---|
| alanine (Ala, A) | arginine (Arg, R) |
| asparagine (Asn, N) | aspartic acid (Asp, D) |
| cysteine (Cys, C) | glutamic acid (Glu, E) |
| glutamine (Gln, Q) | glycine (Gly, G) |
| histidine (His, H) | isoleucine (Ile, I) |
| leucine (Leu, L) | lysine (Lys, K) |
| methionine (Met, M) | phenylalanine (Phe, F) |
| proline (Pro, P) | serine (Ser, S) |
| threonine (Thr, T) | tryptophan (Trp, W) |
| tyrosine (Tyr, Y) | valine (Val, V) |

One aspect of the present invention provides a liquid formulation of a long-acting conjugate of glucagon-like peptide-2 (GLP-2).

Specifically, the present invention provides a liquid formulation of a long-acting conjugate of GLP-2, including:
a long-acting conjugate of GLP-2, in which GLP-2 and an immunoglobulin Fc fragment are linked to each other, in a pharmacologically effective amount, and
a stabilizer including a sugar alcohol, a saccharide, or a combination thereof; and a buffering agent.

As used herein, the term "liquid formulation" refers to a drug formulated into a liquid form and is intended to include all liquid formulations for internal use and formulations for external use.

The liquid formulation of the present invention includes a long-acting conjugate of GLP-2 showing a pharmacological effect, and a substance capable of stably maintaining and/or storing the conjugate showing the pharmacological effect for a certain period of time when it is formulated in a liquid form. Components included in addition to the long-acting conjugate of GLP-2 that exhibit the pharmacological effect of the liquid formulation may be used interchangeably with a stabilizer.

In the liquid formulation of the long-acting conjugate of GLP-2 of the present invention, storage stability is important for ensuring accurate dosage.

The liquid formulation of the present invention includes a long-acting conjugate of GLP-2 and a stabilizer.

As used herein, the term "stabilizer" refers to a substance that stably maintains components such as active ingredients for a specific period of time in a formulation.

The stabilizer of the present invention may be an albumin-free stabilizer, but is not limited thereto. Human serum albumin that can be used as a protein stabilizer is prepared from human blood, and thus can be contaminated with pathogenic virus from human, and gelatin or bovine serum albumin can cause diseases or can cause allergic reactions in some patients. The albumin-free stabilizer does not contain a foreign protein such as human or animal serum albumin or purified gelatin, and thus is not susceptible to viral infection.

In the present invention, the stabilizer specifically refers to a substance that allows the long-acting conjugate of GLP-2 to be stored stably. In the long-acting conjugate of GLP-2, storage stability is important not only to ensure an accurate dosage, but also to inhibit potential generation of antigenic substances from the long-acting conjugate of GLP-2.

The stabilizer may include a sugar alcohol, a saccharide, or a combination thereof; and a buffering agent. Such a liquid formulation may be in the form of a solution capable of stably storing the long-acting conjugate of GLP-2.

The buffering agent included in the liquid formulation of the present invention can maintain the pH of a solution so that the pH of the liquid formulation does not rapidly change so as to make the long-acting conjugate of GLP-2 stable.

The buffering agent may be a pH buffering agent, including phosphoric acid and its conjugate base (*i.e*., alkali salt such as phosphate: sodium phosphate, potassium phosphate, or a hydrogen or dihydrogen salt thereof), citric acid and a salt thereof (*e.g*., sodium citrate), acetic acid and a salt thereof (*e.g*., sodium acetate), or histidine and a salt thereof, and a mixture of these buffering agents may also be used, but the buffering agent is not limited thereto.

The liquid formulation of the present invention may include a buffer solution containing the buffering agent as a solvent of the liquid formulation, and specifically, the buffer solution may be selected from the group consisting of a citrate buffer solution (*e.g*., a sodium citrate buffer solution), an acetate buffer solution (*e.g*., a sodium acetate buffer solution), a phosphate buffer solution (*e.g.*, a sodium phosphate buffer solution), a histidine buffer solution, and a combination thereof. More specifically, the buffer solution may be selected from the group consisting of a citrate buffer solution (*e.g*., a sodium citrate buffer solution), an acetate buffer solution (*e.g.*, a sodium acetate buffer solution), a histidine buffer solution, and a combination thereof, and even more specifically, the buffer solution may be selected from the group consisting of a citrate buffer solution (*e.g*., a sodium citrate buffer solution), an acetate buffer solution (*e.g*., a sodium acetate buffer solution), and a combination thereof, but the buffer solution is not limited thereto.

Additionally, the buffer solution or the buffering agent in the liquid formulation (citric acid and a salt thereof, acetic acid and a salt thereof, histidine and a salt thereof, phosphoric acid and a salt thereof, or a combination thereof) may be contained in a concentration sufficient to maintain a target pH of the liquid formulation. Specifically, the concentration of the buffering agent may be about 2 mM to about 200 mM, more specifically about 5 mM to about 100 mM, about 5 mM to about 80 mM, about 5 mM to about 40 mM, about 10 mM to about 40 mM, about 10 mM to about 30 mM, about 15 mM to about 25 mM, but is not particularly limited thereto.

The pH of the buffer solution or the liquid formulation may be in the range of about 4.0 to about 7.0, specifically about 4.0 to about 6.8, about 4.2 to about 6.6, about 4.3 to about 6.5, and more specifically about 4.5 to about 6.5, about 4.5 to about 6.3, about 4.5 to about 6.0, about 4.8 to about 6.5, about 4.8 to about 6.0, about 5.1 to about 6.0, or about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, or 7.0, but the pH is not limited thereto.

As used herein, the term "about" refers to a range which includes all of ±0.5, ±0.4, ±0.3, ±0.2, ±0.1, *etc.* and includes all of the values that are equivalent or similar to those following the values, but the range is not limited thereto.

Meanwhile, in the preparation of the liquid formulation, the components are dissolved in water (*e.g*., WFI), and the pH of the buffer solution or formulation can be adjusted to a desired pH using HCl and/or NaOH, *etc.,* which is a method already commonly used in the art. Therefore, even if the pH adjuster is not additionally mentioned in the claims, it will be understood by those skilled in the art that the formulation can have an adjusted pH through such a method.

The sugar alcohol included in the stabilizer of the present invention refers to a substance containing a plurality of hydroxyl groups, and is a generic term for substances, in which an aldehyde group and/or a ketone group of a saccharide is substituted with an alcohol group, and can increase the stability of the long-acting conjugate of GLP-2. For example, the sugar alcohol may be one or more selected from the group consisting of mannitol and sorbitol, but is not limited thereto.

The saccharide, which is included in the stabilizer of the present invention, refers to monosaccharides, disaccharides, polysaccharides, oligosaccharides, *etc.,* and can increase the stability of the long-acting conjugate of GLP-2. Specific examples may include monosaccharides such as mannose, glucose, fructose, galactose, fucose, and xylose; disaccharides such as lactose, maltose, and sucrose; and polysaccharides such as raffinose and dextran, but are not limited thereto.

The sugar alcohol, saccharide, or combination thereof may be present in a concentration of about 1% (w/v) to about 20% (w/v), about 1% (w/v) to about 15% (w/v), about 2% (w/v) to about 15% (w/v), about 2% (w/v) to about 12% (w/v), about 2% (w/v) to about 10% (w/v), about 3% (w/v) to about 10% (w/v), about 4% (w/v) to about 10% (w/v), about 4% (w/v) to about 9% (w/v), about 5% (w/v) to about 9% (w/v), about 5% (w/v) to about 8% (w/v), about 1% (w/v), 2% (w/v), 3% (w/v), 4% (w/v), 5% (w/v), 6% (w/v), 7% (w/v), 8% (w/v), 9% (w/v), 10% (w/v), 11% (w/v), 12% (w/v), 13% (w/v), 14% (w/v), 15% (w/v), 16% (w/v), 17% (w/v), 18% (w/v), 19% (w/v), or 20% (w/v), about 5% (w/v), or about 8% (w/v) relative to the total solution of the liquid formulation, but is not particularly limited thereto.

Additionally, the stabilizer of the present invention may further include one or more components selected from the group consisting of a nonionic surfactant and an amino acid, but is not limited thereto. Accordingly, the stabilizer may consist essentially of a sugar alcohol, a saccharide, or a combination thereof; and a buffering agent, and may also consist essentially of (i) a sugar alcohol, a saccharide, or a combination thereof; a buffering agent; and a nonionic surfactant, (ii) a sugar alcohol, a saccharide, or a combination thereof; a buffering agent; and an amino acid, or (iii) a sugar alcohol, a saccharide, or a combination thereof; a buffering agent; a nonionic surfactant; and an amino acid, but is not limited thereto.

Here, it is apparent that all of the contents described above or below apply to the type, concentration, or pH of each component constituting the stabilizer.

Although not particularly limited thereto, the nonionic surfactant lowers the surface tension of the protein solution, thereby preventing protein adsorption or aggregation on the hydrophobic surface.

Specific examples of the nonionic surfactant that can be used in the present invention may include polysorbates or poloxamers, and these can be used one by one or in combinations of two or more thereof.

Specifically, the nonionic surfactant may be polysorbate 80, polysorbate 60, polysorbate 40, polysorbate 20, or poloxamer 188, and these may be used in combination, but is not particularly limited thereto.

In the present invention, it is preferable that the nonionic surfactant is not contained in a high concentration, and specifically, it may be contained in a concentration of about 0.2% (w/v) or less, for example, about 0.001% (w/v) to about 0.2% (w/v), about 0.001% (w/v) to about 0.1% (w/v), about 0.001% (w/v) to about 0.05% (w/v), about 0.005% (w/v) to about 0.08% (w/v), about 0.002% (w/v) to about 0.05% (w/v), about 0.005% (w/v) to about 0.05% (w/v), about 0.01% (w/v) to about 0.05% (w/v), about 0.01% (w/v) to about 0.04% (w/v), about 0.01% (w/v) to about 0.03% (w/v), or about 0.02% (w/v) in the formulation, but the concentration is not particularly limited thereto.

The amino acid may be methionine, arginine, histidine, glycine, cysteine or a combination thereof, and specifically methionine. In addition, the methionine may be L-methionine, but is not particularly limited thereto.

The amino acid may inhibit the generation of impurities that may occur due to the oxidation reaction of the protein, *etc.,* but is not particularly limited thereto.

The amino acid may be present in a concentration of about 0.01 mg/mL to about 1 mg/mL, specifically about 0.01 mg/mL to about 0.8 mg/mL, about 0.01 mg/mL to about 0.5 mg/mL, about 0.02 mg/mL to about 0.5 mg/mL, or about 0.02 mg/mL to about 0.4 mg/mL in the formulation, but is not limited particularly limited thereto.

Meanwhile, the liquid formulation may further include polyhydric alcohol, but is not particularly limited thereto.

For example, the liquid formulation may include polyhydric alcohol as well as a stabilizer including a sugar alcohol, a saccharide, or a combination thereof; and a buffering agent. Specifically, the liquid formulation may further include polyhydric alcohol in the stabilizer consisting essentially of (i) a sugar alcohol, a saccharide, or a combination thereof; a buffering agent; and a nonionic surfactant, (ii) a sugar alcohol, a saccharide, or a combination thereof; a buffering agent; and an amino acid, or (iii) a sugar alcohol, a saccharide, or a combination thereof; a buffering agent; a nonionic surfactant; and an amino acid, but is not particularly limited thereto.

Preferred examples of polyhydric alcohols that may be further included in the liquid formulation of the present invention may include propylene glycol and low-molecular weight polyethylene glycol, glycerol, low-molecular weight polypropylene glycol, *etc.,* and these may be used in one or two or more combinations thereof. In addition, sugar alcohols may be excluded from the polyhydric alcohols of the present invention, but is not limited thereto.

Meanwhile, the liquid formulation of the present invention may optionally further include other components or materials known in the art within the range that does not impair the effects of the present invention, in addition to the sugar alcohol, saccharide, or combination thereof; buffering agent; amino acid; and nonionic surfactant described above.

Meanwhile, in one embodiment, the liquid formulation of the present invention may or may not include an isotonic agent. The isotonic agent refers to a substance that can control osmotic pressure. The isotonic agent may serve to properly maintain osmotic pressure when the liquid formulation according to the present invention is administered to the body.

Representative examples of the isotonic agent may include a water-soluble inorganic salt such as sodium chloride, sodium sulfate, or sodium citrate, specifically sodium chloride, but is not particularly limited thereto. Such an inorganic salt may be an optional component further included in the above-described stabilizer, and is not particularly limited thereto. Additionally, the above-described stabilizer may serve as an isotonic agent.

The concentration of the isotonic agent in the formulation of the present invention may be 0 mM to 200 mM, 0 mM to 150 mM, 0 mM to 100 mM, 10 mM to 200 mM, 10 mM to 150 mM, 10 mM to 100 mM, 10 mM to 50 mM, 20 mM to 100 mM, 20 mM to 80 mM, 20 mM to 50 mM, 20 mM to 30 mM, or 40 mM to 50 mM, but is not particularly limited thereto.

As used herein, the term "long-acting conjugate of GLP-2" is an active ingredient included in the liquid formulation of the present invention, and may be included in the formulation in a pharmacologically effective amount, for example, the concentration of the long-acting conjugate of GLP-2 may be about 18 nmol/mL to about 18,630 nmol/mL, about 18 nmol/mL to about 14,904 nmol/mL, about 18 nmol/mL to about 9,315 nmol/mL, about 18 nmol/mL to about 7,452 nmol/mL, about 18 nmol/mL to about 5,589 nmol/mL, about 18 nmol/mL to about 4,658 nmol/mL, about 18 nmol/mL to about 2,981 nmol/mL, about 18 nmol/mL to about 1,863 nmol/mL, about 18 nmol/mL to about 1,491 nmol/mL, about 18 nmol/mL to about 1,211 nmol/mL, about 18 nmol/mL to about 932 nmol/mL, about 18 nmol/mL to about 746 nmol/mL, about 18 nmol/mL to about 559 nmol/mL, about 18 nmol/mL to about 373 nmol/mL, about 37 nmol/mL to about 5,589 nmol/mL, about 93 nmol/mL to about 2,236 nmol/mL, about 149 nmol/mL to about 1,491 nmol/mL, about 186 nmol/mL to about 2,981 nmol/mL, about 186 nmol/mL to about 1,118 nmol/mL, about 93 nmol/mL to about 18,630 nmol/mL, about 186 nmol/mL to about 9,315 nmol/mL, about 279 nmol/mL to about 5,589 nmol/mL, about 372 nmol/mL to about 4,658 nmol/mL, about 465 nmol/mL to about 3,726 nmol/mL, about 558 nmol/mL to about 2,981 nmol/mL, about 18 nmol/mL to about 14,910 nmol/mL, about 18 nmol/mL to about 9,320 nmol/mL, about 18 nmol/mL to about 7,460 nmol/mL, about 18 nmol/mL to about 5,590 nmol/mL, about 18 nmol/mL to about 4,660 nmol/mL, about 18 nmol/mL to about 1,870 nmol/mL, about 18 nmol/mL to about 1,220 nmol/mL, about 18 nmol/mL to about 940 nmol/mL, about 18 nmol/mL to about 750 nmol/mL, about 18 nmol/mL to about 560 nmol/mL, or about 18 nmol/mL to about 380 nmol/mL, about 37 nmol/mL to about 5,590 nmol/mL, about 93 nmol/mL to about 2,240 nmol/mL, about 149 nmol/mL to about 1,500 nmol/mL, about 186 nmol/mL to about 2,990 nmol/mL, about 186 nmol/mL to about 1,120 nmol/mL, about 186 nmol/mL to about 9,320 nmol/mL, about 279 nmol/mL to about 5,590 nmol/mL, about 372 nmol/mL to about 4,660 nmol/mL, about 465 nmol/mL to about 3,730 nmol/mL, about 558 nmol/mL to about 2,990 nmol/mL, about 186 nmol/mL, about 186.3 nmol/mL, about 187 nmol/mL, about 1,117 nmol/mL, about 1,117.8 nmol/mL, about 1,118 nmol/mL, about 558 nmol/mL, about 558.9 nmol/mL, about 559 nmol/mL, about 1,490 nmol/mL, about 1,490.4 nmol/mL, about 1,491 nmol/mL, about 2,980 nmol/mL, about 2,980.8 nmol/mL, or about 2,981 nmol/mL, but is not limited thereto.

Specifically, the long-acting conjugate may be a form in which an immunoglobulin Fc fragment is linked to GLP-2. The conjugate may exhibit an increased duration of efficacy compared to GLP-2, to which an immunoglobulin is not conjugated, and in the present invention, such a conjugate is referred to as a "long-acting conjugate". In the present invention, the long-acting conjugate may be used interchangeably conjugate.

Meanwhile, the conjugate may be non-naturally occurring.

Additionally, in the long-acting conjugate of GLP-2, the linkage between GLP-2 and the immunoglobulin Fc fragment may be achieved by a physical or chemical bond, a non-covalent or covalent bond, and specifically a covalent bond, but is not limited thereto.

Additionally, the method of linking GLP-2 and the immunoglobulin Fc fragment is not particularly limited, but GLP-2 and the immunoglobulin Fc fragment may be linked to each other through a linker.

Further, Korean Laid-open Patent Publication No. 10-2019-0037181 relating to a long-acting conjugate of GLP-2 is incorporated herein by reference.

In one specific embodiment, the long-acting conjugate of GLP-2 has the structure of Chemical Formula 1 below:

[Chemical Formula 1] X-Lₐ-F

wherein,
X is GLP-2;
L is a linker containing an ethylene glycol repeating unit;
a is 0 or a natural number, with the proviso that when a is 2 or more, each L is independent of each other;
F is an immunoglobulin Fc fragment; and
"-" may be any chemical bond, such as a non-covalent bond or a covalent bond.

In one specific embodiment, the liquid formulation of the present invention may include:
18 nmol/mL to 18,630 nmol/mL of a long-acting conjugate of GLP-2 represented by Chemical Formula 1 above, and
a stabilizer including 1% (w/v) to 20% (w/v) of a sugar alcohol, a saccharide, or a combination thereof; and a buffering agent in an amount for maintaining the pH in the range of 4.5 to 6.5:

In the present specification, the immunoglobulin Fc region encompasses not only a native sequence obtained from papain digestion of an immunoglobulin, but also derivatives thereof, e.g. the sequence different from the native sequence in which one or more amino acid residues in the native sequence are modified by deletion, insertion, non-conservative or conservative substitution, or a combination thereof.

As used herein, the term "glucagon-like peptide-2 (GLP-2)", being a peptide having a function of preventing, treating and improving intestinal damage, intestinal disease, and gastric disease (X in Chemical Formula 1), includes not only native GLP-2, but also agonists and derivatives thereof, *etc.*

The amino acid sequence of the native GLP-2 is as follows:
GLP-2(1-33)
HADGSFSDEMNTILDNLAARDFINWLIQTKITD (SEQ ID NO: 1)

As used herein, the "GLP-2 receptor agonist" refers to a substance that binds to GLP-2 receptor *in vivo* and causes the same or similar physiological activity as native GLP-2. For example, the GLP-2 agonist may include native GLP-2 or a GLP-2 derivative.

As used herein, the "GLP-2 derivative" may be those in which a variation selected from the group consisting of substitution, addition, deletion, modification and a combination thereof has occurred in one or more amino acids in the native GLP-2 sequences, and may include mimics of native GLP-2 having a function of preventing, treating, and/or improving intestinal damage, intestinal disease, and gastric disease. The added amino acid may be a non-natural amino acid (*e.g*., D-amino acid), and substitution of non-natural amino acids other than natural amino acids is also possible. The added amino acid sequence may be derived from native GLP-2, but is not limited thereto. Additionally, the variation of the amino acids in the present invention may refer to those in which some group of an amino acid residue is chemically substituted (*e.g*., *alpha*-methylation, *alpha*-hydroxylation, substitution with an azido group), removed (*e.g*., deamination) and/or modified (*e.g*., *N*-methylation), independently or in combination with substitution, addition, deletion, or combinations thereof in one or more amino acids, but is not limited thereto.

In one specific embodiment, the GLP-2 derivative of the present invention may have a homology of 80%, 85%, 90%, 95%, 96%, 97%, 98%, 99% or more to the native GLP-2 in the amino acid sequence, or may be in the form in which some group of an amino acid residue of GLP-2 is chemically substituted (*e.g*., *alpha*-methylation, *alpha*-hydroxylation, substitution with an azido group), removed (*e.g.*, deamination) and/or modified (*e.g*., *N*-methylation), but is not limited thereto.

Specifically, the agonists and derivatives of native GLP-2 are not limited thereto, but may have a function of preventing, treating and improving intestinal damage, intestinal disease, and gastric disease.

In one specific embodiment, N-terminal amino group in the GLP-2 of the present invention may be substituted, removed, or modified, but is not limited thereto. In order to prevent binding to the N-terminus of GLP-2, which is an important site for the *in vivo* activity of GLP-2, when preparing the long-acting conjugate of GLP-2, the GLP-2 of the present invention may be prepared by a method of removing the alpha amino group of the N-terminal histidine, a method of substituting the N-terminal amino group with a hydroxyl group or a carboxyl group, a method of removing the α-carbon of the N-terminal histidine and the N-terminal amino group bonded to the α-carbon to leave only an imidazo-acetyl functional group, and a method of modifying the N-terminal amino group with two methyl groups, *etc..*

Specifically, the GLP-2 may be imidazoacetyl-deshistidyl-GLP-2 (CA-GLP-2) in which the α-carbon of the histidine residue, which is the first N-terminal amino acid of GLP-2, and the N-terminal amino group bonded to the α-carbon have been removed; desaminohistidyl GLP-2 (DA-GLP-2) in which the N-terminal amino group of GLP-2 has been removed; β-hydroxyimidazopropionyldeshistidyl GLP-2 (HY-GLP-2) in which the N-terminal amino group of GLP-2 is substituted with a hydroxyl group; *N*-dimethylhistidyl GLP-2 (DM-GLP-2) in which the N-terminal amino group of GLP-2 is modified with two methyl groups; or β-carboxyimidazopropionyl-deshistidyl-GLP-2 (CX-GLP-2) in which the N-terminal amino group of GLP-2 is substituted with a carboxyl, but is not limited thereto. In one non-limiting example, the material structure used to prepare the GLP-2 derivative is as follows:

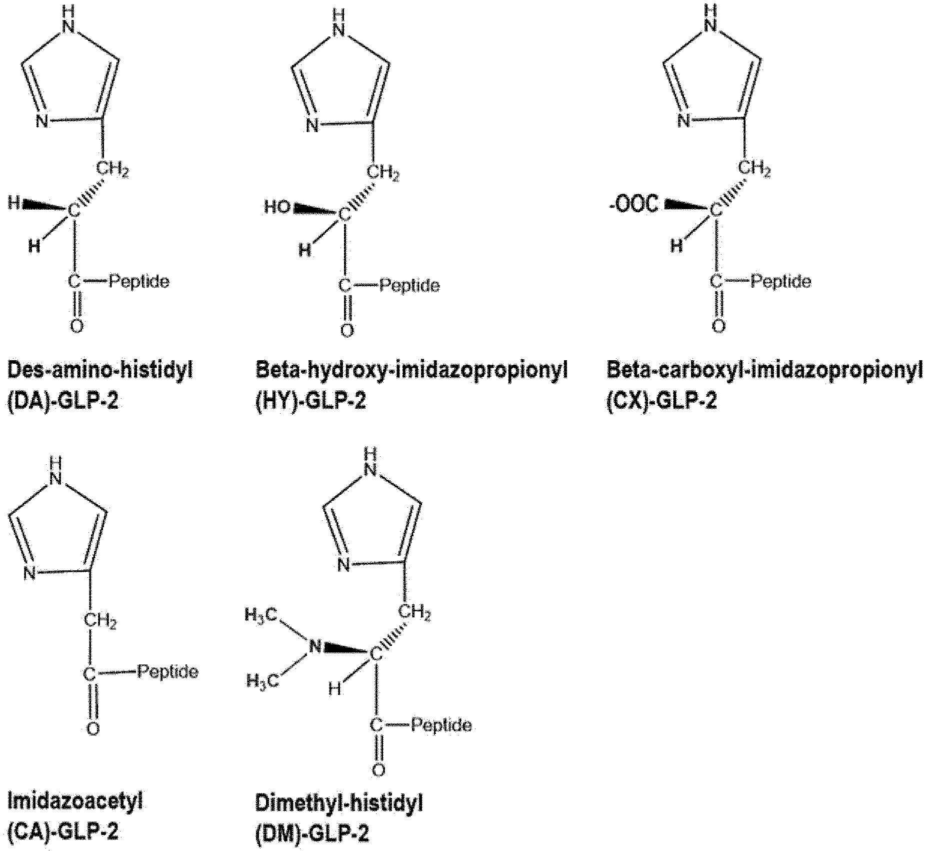

In the present invention, imidazoacetyl-deshistidyl(dine), desaminohistidyl(dine), *beta-*hydroxyimidazopropionyldeshistidyl(dine), *N*-dimethylhistidyl(dine), and *beta*-carboxyimidazopropionyl-deshistidyl(dine) may be used in the same meaning as imidazoacetyl, des-amino-histidyl, *beta*-hydroxy-imidazopropionyl, dimethyl-histidyl, *beta*-carboxyl-imidazopropionyl, respectively.

In one specific embodiment, the GLP-2 derivative may include a variation in one or more amino acids at positions 1, 2, 30, and 33 of SEQ ID NO: 1, but is not limited thereto. Specifically, the variation of the amino acids in the present invention may be selected from the group consisting of substitution, addition, deletion, modification, and combinations thereof in one or more amino acids, wherein the added amino acid may be a non-natural amino acid (*e.g*., D-amino acid), and substitution of non-natural amino acids other than natural amino acids is also possible. The added amino acid sequence may be derived from native GLP-2, but is not limited thereto. Additionally, the variation of the amino acids in the present invention may refer to those in which some group of an amino acid residue is chemically substituted (*e.g*., *alpha*-methylation, *alpha*-hydroxylation, substitution with an azido group), removed (*e.g*., deamination) and/or modified (*e.g*., *N*-methylation), independently or in combination with substitution, addition, deletion, or combinations thereof in one or more amino acids, but is not limited thereto.

In one specific embodiment, the GLP-2 may include an amino acid sequence of General Formula 1 below, but is not limited thereto:

[General Formula 1] X₁X₂DGSFSDEMNTILDNLAARDFINWLIQTX₃₀ITDX₃₄ (SEQ ID NO: 9)

wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent, or lysine, arginine, glutamine, histidine, 6-azidolysine or cysteine.

In one specific embodiment, the GLP-2 may include an amino acid sequence of General Formula 2 below, but is not limited thereto:

[General Formula 2] X₁X₂DGSFSDEMNTILDNLAARDFINWLIQTX₃₀ITDX₃₄ (SEQ ID NO: 10)

wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is any one or more amino acids or any one or more amino acids in which a variation has occurred.

Specifically, the amino acid may be a natural amino acid or a non-natural amino acid, and the variation of the amino acids is as described above.

Additionally, among the amino acid sequences of General Formula 1 or 2, the same sequence as SEQ ID NO: 1 may be excluded, but is not limited thereto.

Specifically, the GLP-2 derivative of the present invention may have a substitution of alanine at the 2^{nd} amino acid of native GLP-2 with glycine or Aib (2-aminoisobutyric acid), a substitution of lysine at the 30^{th} amino acid of native GLP-2 with arginine, or a combination thereof, but is not limited thereto. Additionally, the GLP-2 derivative may be introduced with a thiol group (*e.g*., cysteine), an amino group (*e.g*., lysine, arginine, glutamine or histidine), or an azide group (*e.g*., 6-azidolysine) to the C-terminus (*e.g*., 33^{rd} amino acid), but is not limited thereto.

Since the conjugation occurs in the introduced group when preparing the long-acting conjugate of GLP-2 derivative, these may be used to prepare a GLP-2 conjugate, the binding site of which is selectively controlled. Specifically, the hydroxyl group, thiol group, amino group, or azide group of the GLP-2 derivative may be conjugated to one end of a linker, and a material (*e.g*., an immunoglobulin Fc fragment) capable of increasing the *in vivo* half-life may be conjugated to the other end of the linker. The thiol group, amino group, or azide group may be introduced by adding an amino acid to GLP-2, but is not limited thereto. The thiol group may be introduced by adding cysteine (C) to GLP-2; the amino group may be introduced by adding lysine (K), arginine (R), glutamine (Q), or histidine (H) to GLP-2; and the azide group may be introduced by adding 6-azidolysine (_{AZ}K) to GLP-2, but these are not limited thereto.

Specifically, in the GLP-2 derivative, at least one of the residues may be cysteine, lysine, arginine, glutamine, histidine or 6-azido-lysine, but is not limited thereto.

Specifically, the GLP-2 derivative of the present invention may include the substitution of alanine, which is the 2^{nd} amino acid of native GLP-2, with glycine and the introduction of a thiol group (*e.g*., cysteine) into the C-terminus of the GLP-2; and more specifically, the GLP-2 derivative may include imidazoacetyldeshistidine in which the α-carbon of the histidine residue, which is the first amino acid at the N-terminus of the GLP-2, and the N-terminal amino group bound to the α-carbon are removed, for example, it may have an amino acid sequence of SEQ ID NO: 2, but is not limited thereto.

Specifically, the GLP-2 derivative of the present invention may include the substitution of alanine, which is the 2^{nd} amino acid of native GLP-2, with glycine, and the introduction of an amino group (*e.g*., lysine) into the C-terminus; and more specifically, the GLP-2 derivative may include imidazoacetyldeshistidine in which the α-carbon of the histidine residue, which is the first amino acid at the N-terminus of the GLP-2, and the N-terminal amino group bound to the α-carbon are removed, for example, it may have an amino acid sequence of SEQ ID NO: 3, but is not limited thereto.

Specifically, the GLP-2 derivative of the present invention may include the substitution of alanine, which is the 2^{nd} amino acid of native GLP-2, with glycine, the substitution of lysine, which is the 30^{th} amino acid of native GLP-2, with arginine, and the introduction of an amino group (*e.g*., lysine) into the C-terminus; and more specifically, the GLP-2 derivative may include imidazoacetyldeshistidine in which the α-carbon of the histidine residue, which is the first amino acid at the N-terminus of the GLP-2, and the N-terminal amino group bound to the α-carbon are removed, for example, it may have an amino acid sequence of SEQ ID NO: 4, but is not limited thereto.

Specifically, the GLP-2 derivative of the present invention may include the substitution of alanine, which is the 2^{nd} amino acid of native GLP-2, with glycine, and the introduction of an azide group (*e.g*., 6-azidolysine) into the C-terminus; and more specifically, the GLP-2 derivative may include imidazoacetyldeshistidine in which the α-carbon of the histidine residue, which is the first amino acid at the N-terminus of the GLP-2, and the N-terminal amino group bound to the α-carbon are removed, for example, it may have an amino acid sequence of SEQ ID NO: 5, but is not limited thereto.

Specifically, the GLP-2 derivative of the present invention may include the substitution of alanine, which is the 2^{nd} amino acid of native GLP-2, with glycine, the substitution of lysine, which is the 30^{th} amino acid of native GLP-2, with arginine, and the introduction of a thiol group (*e.g*., cysteine) into the C-terminus; and more specifically, the GLP-2 derivative may include imidazoacetyldeshistidine in which the α-carbon of the histidine residue, which is the first amino acid at the N-terminus of the GLP-2, and the N-terminal amino group bound to the α-carbon are removed, for example, it may have an amino acid sequence of SEQ ID NO: 6, but is not limited thereto.

Specifically, the GLP-2 derivative of the present invention may include the substitution of alanine, which is the 2^{nd} amino acid of native GLP-2, with 2-aminoisobutyric acid, and the introduction of a thiol group (*e.g*., cysteine) into the C-terminus, for example, it may have an amino acid sequence of SEQ ID NO: 8; and more specifically, the GLP-2 derivative may include imidazoacetyldeshistidine in which the α-carbon of the histidine residue, which is the first amino acid at the N-terminus of the GLP-2, and the N-terminal amino group bound to the α-carbon are removed, for example, it may have an amino acid sequence of SEQ ID NO: 7, but is not limited thereto.

The GLP-2 derivatives of SEQ ID NOS: 2 to 8 are shown in Table 1 below.

**[Table 1]**

| **NAME** | SEQUENCE | SEQ ID NO |
|---|---|---|
| CA GLP-2 KC | *_{ca}*HGDGSFSDEMNTILDNLAARDFINWLIQTKITDC | 2 |
| CA GLP-2 KK | *_{ca}*HGDGSFSDEMNTILDNLAARDFINWLIQTKITDK | 3 |
| CA GLP-2 RK | *_{ca}*HGDGSFSDEMNTILDNLAARDFINWLIQTRITDK | 4 |
| CA GLP-2 K_{AZ}K | *_{ca}*HGDGSFSDEMNTILDNLAARDFINWLIQTKITD_{AZ}K | 5 |
| CA GLP-2 RC | *_{ca}*HGDGSFSDEMNTILDNLAARDFINWLIQTRITDC | 6 |
| CA GLP-2 Aib | *_{ca}*HAibDGSFSDEMNTILDNLAARDFINWLIQTKITDC | 7 |
| GLP-2 Aib | HAibDGSFSDEMNTILDNLAARDFINWLIQTKITDC | 8 |

In Table 1 above, _{ca}H indicates imidazoacetyldeshistidine substituted instead of histidine; Aib indicates 2-aminoisobutyric acid; and _{AZ}K indicates 6-azido-L-lysine.

The GLP-2 derivatives according to the present invention may be peptides including the specific sequences described above, or may be peptides consisting (essentially) of the specific sequences described above, but the GLP-2 derivatives are not limited thereto.

Meanwhile, although described as a peptide or a GLP-2 "consisting of a particular SEQ ID NO" in the present invention, such expression does not exclude a meaningless sequence addition upstream or downstream of the amino acid sequence of the corresponding SEQ ID NO, or a naturally occurring mutation therein, or a silent mutation therein, as long as the peptide or GLP-2 having such sequence addition or mutation has an activity the same as or corresponding to the peptide or GLP-2 which consists of an amino acid sequence of the corresponding SEQ ID NO. It is obvious that such peptide or the GLP-2 having the sequence addition or mutation belongs to the scope of the present invention.

Specifically, in General Formula 1 or 2, (1) X₂ may be glycine or Aib, (2) X₃₀ may be lysine or arginine, or (3) X₂ may be glycine or Aib, and X₃₀ may be lysine or arginine, but these are not limited thereto.

Specifically, in General Formula 1 or 2, the GLP-2 derivative may be:
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine, but these are not limited thereto.

In the present invention, the GLP-2 derivatives may be in a modified form where the N-terminus and/or C-terminus, *etc.* thereof is chemically modified or protected by organic groups, or amino acids may be added to the terminus of the GLP-2, for its protection from proteases *in vivo* while increasing its stability.

In particular, in the case of a chemically synthesized peptide, its N- and C-termini are electrically charged. Therefore, in order to remove such charge, the N-terminus of the peptide may be acetylated and/or the C-terminus of the peptide may be amidated, but the peptide modification is not particularly limited thereto. Specifically, in the present invention, the GLP-2 derivatives may be non-modified or amidated at the C-terminus thereof, but the modification is not limited thereto.

The GLP-2 of the present invention can be synthesized through a solid phase synthesis method, can be produced by a recombinant method, and can be produced by commercially.

In Chemical Formula 1 above, F and X may be conjugated to each other through L via a covalent chemical bond, a non-covalent chemical bond, or a combination thereof.

More specifically, X and L, and L and F may be linked to each other via a covalent bond, and in this case, the conjugate may be a conjugate in which X, L, and F are each linked via a covalent bond in the order of Chemical Formula 1.

Additionally, F may be directly linked to X (i.e., a is 0 in Chemical Formula 1) or may be linked via a linker (L).

In one embodiment, Lₐ, which is one component of the long-acting conjugate of Chemical Formula 1, may be a linker containing an ethylene glycol repeating unit (e.g., polyethylene glycol), and additionally, those derivatives which are already known in the art and the derivatives that can easily be prepared at the technological level of those skilled in the art are included in the scope of the present invention.

The L, which is a linker containing an ethylene glycol repeating unit, may include a functional group at an end, which is used in the preparation of the conjugate, before it is formed into a conjugate. The long-acting conjugate according to the present invention may be in the form in which X and F are linked through the functional group, but is not limited thereto. In the present invention, the linker containing an ethylene glycol repeating unit may include two, or three or more functional groups, and each functional group may be the same as or different from each other, but is not limited thereto.

Specifically, the linker may be polyethylene glycol (PEG) represented by Chemical Formula 4 below, but the linker is not limited thereto: 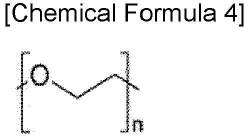 wherein n is 10 to 2,400, n is 10 to 480, or n is 50 to 250, but the range of n is not limited thereto.

In the long-acting conjugate above, the PEG moiety may include not only the -(CH₂CH₂O)ₙ- structure, but also an oxygen atom interposed between a linking element and the -(CH₂CH₂O)ₙ- structure, but the PEG moiety is not limited thereto.

Additionally, in a specific embodiment, the conjugate may have a structure in which an immunoglobulin fragment (F) is linked to GLP-2 by a covalent bond through a linker containing an ethylene glycol repeating unit, but the structure of the conjugate is not limited thereto.

The polyethylene glycol is a general term including all of the forms of homopolymers of ethylene glycol, PEG copolymers, and monomethyl-substituted PEG polymers (mPEG), but the polyethylene glycol is not particularly limited thereto.

In one embodiment, the ethylene glycol repeating unit may be represented by, for example, [OCH₂CH₂]ₙ, and the value of n is a natural number and the average molecular weight of the [OCH₂CH₂]ₙ region in the peptide conjugate, for example, the number average molecular weight, may be determined to be greater than 0 to about 100 kDa, but is not limited thereto. In another example, the value of n is a natural number, and the average molecular weight of the [OCH₂CH₂]ₙ region in the peptide conjugate, for example, the number average molecular weight, may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about 1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, about 3.4 kDa or about 10 kDa, but is not limited thereto.

In one specific embodiment, both ends of the linker may be conjugated to a thiol group, an amino group, or a hydroxyl group of the immunoglobulin Fc region, and may be conjugated to a thiol group, an amino group, an azide group, or a hydroxyl group of the GLP-2, but is not limited thereto.

Specifically, the linker may include a reactive group capable of binding to each of the immunoglobulin Fc and GLP-2 at both ends. Specifically, the linker may include a reactive group that can bind to a thiol group of cysteine; an amino group located at the N-terminus, lysine, arginine, glutamine, and/or histidine; and/or a hydroxyl group located at the C-terminus in the immunoglobulin Fc fragment, and a reactive group that can bind to a thiol group of cysteine; an amino group of lysine, arginine, glutamine, and/or histidine; an azide group of azido-lysine; and/or a hydroxyl group in the GLP-2, but the reactive groups are not limited thereto.

More specifically, the reactive group may be one or more selected from the group consisting of an aldehyde group, a maleimide group, and a succinimide derivative, but is not limited thereto.

In the above, as an example of the aldehyde group, a propionaldehyde group or butyraldehyde group may be used, but the aldehyde group is not limited thereto.

In the above, as a succinimide derivative, succinimidyl carboxymethyl, succinimidyl valerate, succinimidyl methylbutanoate, succinimidyl methylpropionate, succinimidyl butanoate, succinimidyl propionate, *N*-hydroxysuccinimide, hydroxy succinimidyl, or succinimidyl carbonate may be used, but the succinimide derivative is not limited thereto.

The linker may be linked to F, the immunoglobulin Fc, and X, the GLP-2, through the reactive groups to be converted to a linker moiety.

Additionally, the final product produced through reductive amination by an aldehyde bond is much more stable than that linked by an amide bond. The aldehyde reactive group selectively reacts at the N-terminus at a low pH, while it can form a covalent bond with a lysine residue at a high pH (e.g., pH 9.0).

The terminal reactive groups of the linker of the present invention may be the same as or different from each other. The linker may have an aldehyde reactive group at both ends. Alternatively, the linker may have an aldehyde group and a maleimide group at each end, or may have an aldehyde group and a succinimide reactive group at each end, but is not limited thereto.

For example, the linker may have a maleimide group at one end and an aldehyde group, a propionaldehyde group, or a butyraldehyde group at the other end. As another example, the linker may have a succinimidyl group at one end and a propionaldehyde group or butyraldehyde group at the other end.

When a polyethylene glycol having a hydroxy reactive group at propionaldehyde end is used as a linker, the conjugate of the present invention may be prepared by activating the hydroxy group to various reactive groups according to known chemical reactions or by using a commercially available polyethylene glycol having a modified reactive group.

In a specific embodiment, the reactive group of the linker may be linked to a cysteine residue of GLP-2, more specifically to the -SH group of cysteine, but is not limited thereto.

When maleimide-PEG-aldehyde is used, the maleimide group may be linked to the -SH group of GLP-2 by a thioether bond, and the aldehyde group may be linked to the -NH₂ group of the immunoglobulin Fc through reductive alkylation, but is not limited thereto, and this is merely one embodiment.

Through the reductive alkylation, a structure such as - PEG-O-CH₂CH₂CH₂NH-immunoglobulin Fc may be formed by linking an amino group at the N-terminus of an immunoglobulin Fc fragment to an oxygen atom located at one end of the PEG through a linker reactive group having a structure of - CH₂CH₂CH₂-; and a structure, in which one end of the PEG is linked to a sulfur atom located at the cysteine of GLP-2 through a thioether bond, may be formed. The thioether bond described above may include the following structure:

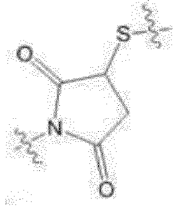

However, the linker is not particularly limited to the above embodiment, and it is merely one embodiment.

Additionally, in the above conjugate, the reactive group of the linker may be linked to -NH₂ located at the N-terminus of an immunoglobulin Fc fragment, but this is merely one embodiment.

In addition, in the conjugate, the GLP-2 may be linked to a linker having a reactive group through the C-terminus, but this is merely one embodiment.

As used herein, the term "C-terminus" refers to a carboxy terminus of a peptide, and refers to a position capable of binding with the linker for the purpose of the present invention. For example, the C-terminus may include the amino acid residue at the most terminal end of the C-terminus as well as amino acid residues near the C-terminus, and specifically may include the 1^{st} to 20^{th} amino acid residues from the most terminal end, but the C-terminus is not limited thereto.

In one embodiment, the conjugate of Chemical Formula 1 may have a structure of Chemical Formula 2 or 3 below:

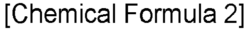

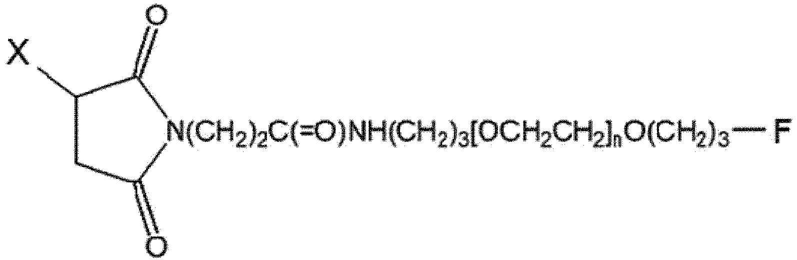

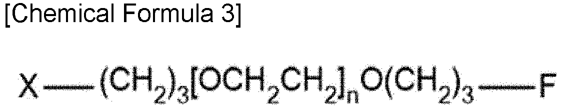

In Chemical Formula 2 or 3, X is the peptide of Chemical Formula 1 described above;
F is an immunoglobulin Fc fragment; and
n may be a natural number. In particular, the description of n is the same as described above.

In one embodiment, the long-acting conjugate of Chemical Formula 2 has a structure in which the X, which is the GLP-2, and F, which is the immunoglobulin Fc fragment, are covalently linked through an ethylene glycol repeating unit, wherein each X may be linked to a succinimide ring of Chemical Formula 2, and F may be linked to an oxypropylene group of Chemical Formula 2. Additionally, the long-acting conjugate of Chemical Formula 3 has a structure in which the X, which is the GLP-2, and F, which is the immunoglobulin Fc fragment, are covalently linked through an ethylene glycol repeating unit, wherein each X may be linked to an oxypropylene group of Chemical Formula 3, and F may be linked to other oxypropylene group of Chemical Formula 3.

In Chemical Formula 2 or 3 above, the value of n may be determined such that the average molecular weight of the [OCH₂CH₂]ₙ region in the peptide conjugate, for example, the number average molecular weight, is 1 kDa to 100 kDa, or 1 kDa to 20 kDa, or 10 kDa, but is not limited thereto.

In one embodiment, the succinimide ring of Chemical Formula 2 or the moiety, at which X is linked to the succinimide ring of Chemical Formula 2, may be a sulfur atom of the C-terminal cysteine of X. Additionally, the oxypropylene group of Chemical Formula 3 or the moiety, at which X is linked to the oxypropylene group of Chemical Formula 3, may be a sulfur atom of the C-terminal cysteine of X.

In F, the moiety linked to the oxypropylene group of Chemical Formula 2 or 3 is not specifically limited. In one embodiment of the present invention, the moiety of F linked to the oxypropylene group may be an N-terminal nitrogen or a nitrogen atom of an internal residue of F (*e.g*., epsilon nitrogen of lysine). In one specific embodiment of the present invention, the moiety where F is linked to the oxypropylene group of Chemical Formula 2 or 3 may be the N-terminal proline of F, but is not limited thereto.

As used herein, the term "immunoglobulin Fc fragment" refers to a heavy chain constant region excluding the heavy and light chain variable regions of an immunoglobulin. Specifically, the immunoglobulin Fc fragment may include the heavy chain constant region 2 (CH2) and/or heavy chain constant region 3 (CH3) portions, and more specifically may further include a hinge region (all or part of the hinge region). In the present invention, the immunoglobulin Fc fragment may be used interchangeably with the immunoglobulin Fc region.

The immunoglobulin Fc region in the present specification encompasses not only a native sequence obtained from papain digestion of an immunoglobulin, but also derivatives, substituents thereof, for example, variants, in which one or more amino acid residues in the native sequence are modified by deletion, insertion, non-conservative or conservative substitution, or a combination thereof, and thus the sequence become different from the native sequence, *etc.,* provided that the above derivatives, substituents, and variants retain FcRn binding ability.

The immunoglobulin Fc fragment may be one constitution constituting the moiety of the conjugate of Chemical Formula 1 of the present invention. Specifically, it may correspond to F in the Chemical Formulae 1 to 3 above.

The F may have a structure in which two polypeptide chains are linked by an inter-disulfide bond, and the F is linked through a nitrogen atom in only one of the two chains, but is not limited thereto. The linkage through the nitrogen atom may be linkage to the epsilon amino atom of lysine or the N-terminal amino group by reductive amination.

The reductive amination reaction refers to a reaction in which an amine group or amino group of a reactant reacts with an aldehyde (i.e., a functional group capable of reductive amination) of another reactant to produce an amine, and thereafter, an amine bond is formed by a reduction reaction. The reductive amination reaction is a reaction of organic synthesis widely known in the art.

In one embodiment, the F may be linked through the nitrogen atom of the N-terminal proline thereof, but is not limited thereto

Such an immunoglobulin Fc fragment may include a hinge region in the heavy chain constant region, but is not limited thereto.

In the present invention, the immunoglobulin Fc fragment may include a specific hinge sequence in the N-terminus.

As used herein, the term "hinge sequence" refers to a region which is located in the heavy chain and forms a dimer of immunoglobulin Fc fragments through an inter-disulfide bond.

In the present invention, the hinge sequence may be modified such that a part of the hinge sequence having the following amino acid sequence is deleted and thus there is only one cysteine residue in the sequence, but is not limited thereto:
Glu-Ser-Lys-Tyr-Gly-Pro-Pro-Cys-Pro-Ser-Cys-Pro (SEQ ID NO: 11).

The hinge sequence may be one in which the 8^{th} or 11^{th} cysteine residue in the hinge sequence of SEQ ID NO: 11 is deleted and thus only one cysteine residue is included in the sequence. The hinge sequence of the present invention may consist of 3 to 12 amino acids, including only one cysteine residue, but the hinge sequence is not limited thereto. More specifically, the hinge sequence of the present invention may have the following sequences: Glu-Ser-Lys-Tyr-Gly-Pro-Pro-Pro-Ser-Cys-Pro (SEQ ID NO: 12), Glu-Ser-Lys-Tyr-Gly-Pro-Pro-Cys-Pro-Ser-Pro (SEQ ID NO: 13), Glu-Ser-Lys-Tyr-Gly-Pro-Pro-Cys-Pro-Ser (SEQ ID NO: 14), Glu-Ser-Lys-Tyr-Gly-Pro-Pro-Cys-Pro-Pro (SEQ ID NO: 15), Lys-Tyr-Gly-Pro-Pro-Cys-Pro-Ser (SEQ ID NO: 16), Glu-Ser-Lys-Tyr-Gly-Pro-Pro-Cys (SEQ ID NO: 17), Glu-Lys-Tyr-Gly-Pro-Pro-Cys (SEQ ID NO: 18), Glu-Ser-Pro-Ser-Cys-Pro (SEQ ID NO: 19), Glu-Pro-Ser-Cys-Pro (SEQ ID NO: 20), Pro-Ser-Cys-Pro (SEQ ID NO: 21), Glu-Ser-Lys-Tyr-Gly-Pro-Pro-Ser-Cys-Pro (SEQ ID NO: 22), Lys-Tyr-Gly-Pro-Pro-Pro-Ser-Cys-Pro (SEQ ID NO: 23), Glu-Ser-Lys-Tyr-Gly-Pro-Ser-Cys-Pro (SEQ ID NO: 24), Glu-Ser-Lys-Tyr-Gly-Pro-Pro-Cys (SEQ ID NO: 25), Lys-Tyr-Gly-Pro-Pro-Cys-Pro (SEQ ID NO: 26), Glu-Ser-Lys-Pro-Ser-Cys-Pro (SEQ ID NO: 27), Glu-Ser-Pro-Ser-Cys-Pro (SEQ ID NO: 28), Glu-Pro-Ser-Cys (SEQ ID NO: 29), Ser-Cys-Pro (SEQ ID NO: 30).

More specifically, the hinge sequence may include the amino acid sequence of SEQ ID NO: 30 (Ser-Cys-Pro) or SEQ ID NO: 21 (Pro-Ser-Cys-Pro), but is not limited thereto.

In a more specific embodiment of the long-acting conjugate of GLP-2 derivatives of the present invention, the N-terminal of the immunoglobulin Fc region in the conjugate is proline, and in this conjugate, the Fc region is linked to a linker through the nitrogen atom of proline.

In one specific embodiment, the immunoglobulin Fc fragment may be in a form in which two molecules of the immunoglobulin Fc chain form a dimer of homodimer or heterodimer due to the presence of a hinge sequence therein, and in addition, the long-acting conjugate of the present invention may be in a form in which one end of the linker is linked to one chain of the dimeric immunoglobulin Fc fragments, but the immunoglobulin Fc fragment and the conjugate are not limited thereto.

In addition, the immunoglobulin Fc fragment of the present invention may include a hinge sequence in the N-terminus, but is not limited thereto.

As used herein, the term "N-terminus" refers to the amino terminus of a protein or polypeptide, and it may include 1, 2, 3, 4, 5, 6, 7, 8, 9, or 10 or more amino acids from the most terminal end or the most terminal end of the amino terminus.

Additionally, the immunoglobulin Fc fragment of the present invention may be an extended Fc fragment, which includes all or part of the heavy chain constant region 1 (CH1) and/or light chain constant region 1 (CL1) excluding the heavy chain and light chain variable regions of an immunoglobulin, as long as it has substantially the equivalent or an improved effect compared to its native type. Additionally, the immunoglobulin Fc fragment of the present invention may be a region in which some fairly long amino acid sequences corresponding to CH2 and/or CH3 are removed.

For example, the immunoglobulin Fc fragment of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of (i) one or two or more domains among a CH1 domain, a CH2 domain, a CH3 domain, and a CH4 domain and (ii) an immunoglobulin hinge region (or part of the hinge region); or 6) a dimer of each domain of the heavy chain constant region and a light chain constant region, but the immunoglobulin Fc region is not limited thereto.

Additionally, in one embodiment, the immunoglobulin Fc fragment may have a dimeric form, and one molecule of X may be covalently linked to one Fc fragment in a dimeric form, in particular, the immunoglobulin Fc and X may be covalently linked to each other through a linker. Meanwhile, it is also possible that two molecules of X are symmetrically linked to one Fc fragment in a dimeric form. In particular, the immunoglobulin Fc and X may be linked to each other through a linker, but is not limited to the embodiments described above.

In addition, the immunoglobulin Fc fragment of the present invention includes native amino acid sequences as well as sequence derivatives thereof. The amino acid sequence derivative means that the sequence is different from the native amino acid sequence due to deletion, insertion, non-conservative or conservative substitution, or a combination thereof in one or more amino acid residues in the native amino acid sequence.

For example, amino acid residues at positions 214 to 238, 297 to 299, 318 to 322, or 327 to 331 in IgG Fc, which are known to be important for linkage, may be used as the sites suitable for variation.

Additionally, various types of derivatives are available, for example, one where the site capable of forming an inter-disulfide bond is removed; one where several N-terminal amino acids from native Fc are removed; one where a methionine residue is added to the N-terminus of native Fc, *etc.* Additionally, complement binding sites (*e.g*., C1q binding sites) or antibody-dependent cell-mediated cytotoxicity (ADCC) sites may be removed to eliminate the effector function. The techniques for preparing the sequence derivatives of these immunoglobulin Fc fragments are disclosed in International Publication Nos. WO 97/34631, WO 96/32478, *etc.*

Amino acid substitutions in a protein or peptide that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, *etc.*

The Fc derivatives described above may be those which exhibit the same biological activity as the Fc fragment of the present invention and have an increased structural stability of the Fc fragment against heat, pH, *etc.*

Additionally, such an Fc fragment may be obtained from a native type isolated from humans or animals (*e.g*., cows, goats, pigs, mice, rabbits, hamsters, rats, guinea pigs, *etc.*) or may be recombinants or derivatives thereof obtained from transformed animal cells or microorganisms. In particular, the Fc fragment may be obtained from a native immunoglobulin by isolating a whole immunoglobulin from a living human or animal and treating the isolated immunoglobulin with protease. When the whole immunoglobulin is treated with papain, it is cleaved into Fab and Fc fragments, whereas when treated with pepsin, it is cleaved into pF'c and F(ab)₂ fragments. Fc or pF'c can be isolated using size-exclusion chromatography, *etc.* In a more specific embodiment, the Fc fragment may be a recombinant immunoglobulin Fc fragment where a human-derived Fc fragment is obtained from a microorganism.

Additionally, the immunoglobulin Fc fragment may be in the form of native glycans, increased or decreased glycans compared to the native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc fragment, in which the glycans are removed from the Fc, shows a significant decrease in binding affinity for the complement (C1q) and a decrease or removal of antibody-dependent cytotoxicity or complement-dependent cytotoxicity, and thus it does not induce unnecessary immune responses *in vivo.* In this regard, an immunoglobulin Fc fragment in a deglycosylated or aglycosylated form may be more suitable to meet the original object of the present invention as a drug carrier.

As used herein, the term "deglycosylation" refers to an Fc fragment in which glycans are removed with an enzyme, and the term "aglycosylation" refers to a non-glycosylated Fc fragment produced in prokaryotes, more specifically *E*. *coli.*

Meanwhile, the immunoglobulin Fc fragment may be derived from humans or animals (*e.g*., cows, goats, pigs, mice, rabbits, hamsters, rats, guinea pigs, *etc.*), and in a more specific embodiment, it may be derived from humans.

Additionally, the immunoglobulin Fc fragment may be derived from IgG, IgA, IgD, IgE, IgM, or a combination or hybrid thereof. In a more specific embodiment, the immunoglobulin Fc fragment may be derived from IgG or IgM, which are among the most abundant proteins in human blood, and in an even more specific embodiment, it may be derived from IgG, which is known to enhance the half-lives of ligand binding proteins. In an even yet more specific embodiment, the immunoglobulin Fc fragment may be an IgG4 Fc fragment, and in the most specific embodiment, it may be an aglycosylated Fc fragment derived from a human IgG4, but is not limited thereto.

Additionally, in one embodiment, the immunoglobulin Fc fragment, being a human IgG4 Fc fragment, may be in the form of a homodimer in which two monomers are linked through an inter-disulfide bond (an inter-chain form) between cysteines, which are the 3^{rd} amino acid of each monomer. In particular, the homodimer may have two disulfide bonds (an intra-chain form), *i.e*., an intra-disulfide bond between the cysteines at positions 35 and 95 in one monomer and an internal disulfide bond between the cysteines at positions 141 and 199 in other monomer; and/or each monomer may consist of 221 amino acids, and the amino acids forming the homodimer may consist of a total of 442 amino acids, but the number of amino acids is not limited thereto. Specifically, the immunoglobulin Fc fragment may be a homodimer including the amino acid sequence of SEQ ID NO: 32 (consisting of 442 amino acids), in which two of a monomer having the amino acid sequence of SEQ ID NO: 31 (consisting of 221 amino acids) form the homodimer through an inter-disulfide bond between cysteines, which are the 3^{rd} amino acid of each monomer, and in which the monomers of the homodimer independently form an intra-disulfide bond between the cysteines at positions 35 and 95 and an intra-disulfide bond between the cysteines at positions 141 and 199, but the immunoglobulin Fc fragment is not limited thereto.

Meanwhile, as used herein, the term "combination" means that polypeptide encoding single-chain immunoglobulin Fc fragment of the same origin is linked to a single-chain polypeptide of a different origin to form a dimer or multimer. That is, it is possible to prepare a dimer or multimer from two or more fragments selected from the group consisting of IgG Fc fragment, IgA Fc fragment, IgM Fc fragment, IgD Fc fragment, and IgE Fc fragment.

As used herein, the term "hybrid" means that sequences corresponding two or more immunoglobulin Fc fragments of different origins are present in a single-chain of an immunoglobulin constant region. In the present invention, various hybrid forms are possible. For example, the hybrid domain may be composed of one to four domains selected from the group consisting of CH1, CH2, CH3, and CH4 of IgG Fc, IgM Fc, IgA Fc, IgE Fc, and IgD Fc, and may further include a hinge region.

Meanwhile, IgG may also be divided into the IgG1, IgG2, IgG3, and IgG4 subclasses, and the present invention may include combinations or hybrids thereof. Preferred are the IgG2 and IgG4 subclasses, and most preferred is the Fc fragment of IgG4, which rarely has effector functions such as complement-dependent cytotoxicity (CDC).

Meanwhile, the GLP-2 or the long-acting conjugate of GLP-2 may include all of those in the form of the peptide itself, a salt thereof (*e.g*., a pharmaceutically acceptable salt of the peptide), or a solvate thereof.

Additionally, the GLP-2 or the long-acting conjugate of GLP-2 may be in any pharmaceutically acceptable form.

The kind of the salt is not particularly limited. However, the salt is preferably one that is safe and effective to a subject (*e.g*., a mammal), but is not particularly limited thereto.

The term "pharmaceutically acceptable" refers to a material which can be effectively used for the intended use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic acids, organic acids, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc.*; alkali earth metals such as magnesium; and ammonium, *etc.*

As used herein, the term "solvate" refers to a complex formed between the peptide according to the present invention or a salt thereof and a solvent molecule.

In a specific example, the liquid formulation of the present invention may include:
a long-acting conjugate of GLP-2;
a buffering agent selected from the group consisting of citric acid and a salt thereof, acetic acid and a salt thereof, histidine and a salt thereof, phosphoric acid and a salt thereof, and a combination thereof;
glucose, fructose, galactose, lactose, maltose, sucrose, mannitol, sorbitol, or a combination thereof;
a nonionic surfactant selected from the group consisting of poloxamer, polysorbate, and a combination thereof; and
an amino acid selected from the group consisting of methionine, arginine, histidine, glycine, cysteine, and a combination thereof, and
wherein the pH of the liquid formulation is 4.5 to 6.5, but is not limited thereto.

In a specific example, the liquid formulation of the present invention may include:
a long-acting conjugate of GLP-2 in a pharmacologically effective amount (e.g., 18 nmol/mL to 18,630 nmol/mL);
5 mM to 100 mM of a buffering agent;
1% (w/v) to 20% (w/v) of a sugar alcohol, a saccharide, or a combination thereof;
0.001% (w/v) to 0.2% (w/v) of a nonionic surfactant; and
0.01 mg/mL to 1 mg/mL of an amino acid, and
wherein the pH of the liquid formulation is 4.5 to 6.5, but is not limited thereto.

In a specific example, the liquid formulation of the present invention may include:
186 to 18,630 nmol/mL of a long-acting conjugate of GLP-2;
5 mM to 100 mM of a buffering agent of acetic acid and a salt thereof, or citric acid and a salt thereof;
1% (w/v) to 20% (w/v) of sucrose, mannitol, sorbitol, or a combination thereof;
0.001% (w/v) to 0.2% (w/v) of polysorbate; and
0.01 mg/mL to 1 mg/mL of methionine, and
wherein the pH of the liquid formulation is 4.5 to 6.5, but is not limited thereto.

In a specific example, the liquid formulation of the present invention may include:
186 nmol/mL to 2,981 nmol/mL of a long-acting conjugate of GLP-2;
10 mM to 40 mM of a buffering agent of acetic acid and a salt thereof, or citric acid and a salt thereof;
2% (w/v) to 15% (w/v) of sucrose, mannitol, sorbitol, or a combination thereof;
0.002% (w/v) to 0.05% (w/v) of polysorbate; and
0.02 mg/mL to 0.5 mg/mL of methionine, and
wherein the pH of the liquid formulation is 4.8 to 6.0, but is not limited thereto.

Another aspect for implementing the present invention provides a method for preparing the liquid formulation.

Specifically, the method for preparing the liquid formulation may include: mixing (i) the long-acting conjugate of GLP-2, in which GLP-2 and an immunoglobulin Fc fragment are linked to each other; with (ii) a stabilizer including a sugar alcohol, a saccharide, or a combination thereof; and a buffering agent.

The long-acting conjugate of GLP-2, buffering agent, sugar alcohol, saccharide, stabilizer, and liquid formulation are the same as described above.

The treatment method of the present invention may include administering the liquid formulation in a pharmaceutically (therapeutically) effective amount. An appropriate total daily dose of the liquid formulation may be determined within the scope of correct medical judgment by a practitioner, and the liquid formulation may be administered once or several times in divided doses. However, for the purpose of the present invention, it is preferred that the specific pharmaceutically (therapeutically) effective dose of the liquid formulation for any particular patient be applied differently depending on the kind and degree of responses to be achieved, specific compositions including whether other agents are occasionally used therewith, the patient's age, body weight, general health conditions, sex and diet, administration time, administration route, excretion rate of the composition, duration of treatment, various factors including drugs used in combination or simultaneously with the specific compositions, and similar factors well known in the medical field.

Hereinafter, the present invention will be described in more detail with reference to the following Examples. However, these Examples are for illustrative purposes only and the scope of the invention is not limited by these Examples.

### Example 1: Preparation of CA-GLP-2 KC-PEG(10K)-Immunoglobulin Fc Conjugate or CA-GLP-2 RC-PEG(10K)-Immunoglobulin Fc Conjugate

For PEGylation of 10 kDa MAL-ALD PEG (maleimide-PEG-aldehyde, which is a polyethylene glycol having a molecular weight of 10 kDa, wherein the hydrogen at each terminus is modified with a 3-[(3-*N*-maleimidyl)propanoyl]aminopropyl group and a 3-oxopropyl group (propylaldehyde group), respectively; NOF Inc., Japan) to the 34^{th} cysteine residue of the CA-GLP-2 KC or CA-GLP-2 RC (CPC, Chinese Peptide Co, China), the reaction was carried out for 1 to 3 hours with the molar ratio of CA-GLP-2 KC or CA-GLP-2 RC to PEG as 1:1 to 2 and the peptide concentration of 1 mg/mL to 3 mg/mL. Herein, the reaction was carried out in a mixed solvent of 50 mM Tris (pH 7.5) and isopropanol. From the reaction solution, the mono-PEGylated CA-GLP-2 KC or mono-PEGylated CA-GLP-2 RC was purified using an SP Sepharose High Performance column (GE, U.S.A.) utilizing a concentration gradient of potassium chloride and a buffer containing sodium citrate (pH 2.0) and ethanol.

Thereafter, the reaction was carried out at 2°C to 8°C for 12 to 20 hours, with the molar ratio of the purified mono-PEGylated CA-GLP-2 KC or mono-PEGylated CA-GLP-2 RC and the immunoglobulin Fc fragment of SEQ ID NO: 32 as 1:2 to 1:6 and the total protein concentration of 30 mg/mL to 35 mg/mL. Herein, the reaction solution contained a 100 mM potassium phosphate buffer (pH 6.0) and isopropanol in which 20 mM sodium cyanoborohydride (NaCNBH₃) was added as a reducing agent.

Upon completion of the reaction, (i) the long-acting conjugate of CA-GLP-2 KC(10K PEG) derivative (CA-GLP-2 KC-PEG(10K)-immunoglobulin Fc) and (ii) the long-acting conjugate of CA-GLP-2 RC(10K PEG) derivative (CA-GLP-2 RC-PEG(10K)-immunoglobulin Fc), in which the CA-GLP-2 KC or CA-GLP-2 RC is covalently linked to the immunoglobulin Fc by the PEG, were purified from the reaction solution by applying to a Source15Q column (GE, U.S.A.) using the concentration gradient of a bis-Tris buffer (pH 6.5) and sodium chloride, and by applying to a Source 15ISO column (GE, U.S.A.) using the concentration gradient of ammonium sulfate and sodium citrate (pH 5.0 to pH 5.2). As a result of HPLC reverse-phase analysis, the purity of the conjugates was determined to be 92.9% and 95.6%, respectively.

### Example 2: Preparation of CA-GLP-2 RK-PEG(3.4K or 10K)-Immunoglobulin Fc Conjugate

For PEGylation of 3.4 kDa or 10 kDa ALD(2) PEG (aldehyde-PEG-aldehyde, which is a polyethylene glycol having a molecular weight of 3.4 kDa, wherein the hydrogens at each terminus are modified with 3-oxopropyl groups (propylaldehyde groups); NOF Inc., Japan) to the 34^{th} lysine residue of the CA-GLP-2 RK (CPC, Chinese Peptide Co., China), the reaction was carried out at 2°C to 8°C for 4 to 16 hours with the molar ratio of CA-GLP-2 RK to PEG as 1:5 to 1:20 and the peptide concentration of 5 mg/mL to 10 mg/mL. Herein, the reaction was carried out in 20 mM HEPES (pH 7.5) and ethanol, and was performed by adding 20 mM sodium cyanoborohydride as a reducing agent. From the reaction solution, the mono-PEGylated CA-GLP-2 RK was purified by using a Source 15S column (GE, U.S.A.) utilizing a concentration gradient of potassium chloride and a buffer containing sodium citrate (pH 2.0) and ethanol.

Thereafter, the conjugate of the purified mono-PEGylated CA-GLP-2 RK and the immunoglobulin Fc of SEQ ID NO: 32 was prepared and purified according to the same reaction and purification conditions as in Example 1. As a result of HPLC reverse-phase analysis, the purity of (i) the long-acting conjugate of CA-GLP-2 RK(3.4K PEG) derivative (CA-GLP-2 RK-PEG(3.4K)-immunoglobulin Fc) and (ii) the long-acting conjugate of CA GLP-2 RK(10K PEG) derivative (CA-GLP-2 RK-PEG(10K)-immunoglobulin Fc), in which the CA-GLP-2 RK is covalently linked to the immunoglobulin Fc by PEG, was determined to be 94.3% and 92.6%, respectively.

### Example 3: Preparation of CA-GLP-2 KK-PEG(10K)-Immunoglobulin Fc Conjugate and CA-GLP-2 K_{AZ}K-PEG(10K)-Immunoglobulin Fc Conjugate

By using CA-GLP-2 KK and CA-GLP-2 K_{AZ}K according to the method of Example 2, the long-acting conjugate of CA GLP-2 KK(10K PEG) derivative (CA-GLP-2 KK-PEG(10K)-immunoglobulin Fc) and the long-acting conjugate of CA GLP-2 K_{AZ}K(10K PEG) derivative (CA-GLP-2 K_{AZ}K-PEG(10K)-immunoglobulin Fc), in which CA-GLP-2 KK or CA-GLP-2 K_{AZ}K is covalently linked to the immunoglobulin Fc of SEQ ID NO: 32 by PEG, were prepared and purified.

### Example 4: Evaluation of Stability of Long-Acting Conjugate of GLP-2 Derivative Peptide According to Types of Buffering agents and pH

The stability of the long-acting conjugate of GLP-2 derivative peptide (hereinafter referred to as "GLP-2 derivative") according to the types of buffering agents and pH was compared based on the liquid formulation consisting of buffering agents, polysorbate 20 as surfactant, a sugar alcohol, and methionine.

A liquid formulation of the long-acting conjugate of GLP-2 derivative (CA-GLP-2 RK(3.4K PEG)) was prepared using the composition shown in Table 2 (the concentration of the long-acting conjugate in the liquid formulation is about 186.3 nmol/mL) and stored at 25°C for 5 weeks, and then was analyzed by ion-exchange high performance liquid chromatography (IE-HPLC), reverse-phase-high-performance liquid chromatography (RP-HPLC), and size-exclusion chromatography (SE-HPLC).

In Table 3, IE-HPLC (%), RP-HPLC (%), and SE-HPLC (%) indicate Area% / Start Area% (%), indicating the residual ratio of the long-acting conjugate of GLP-2 derivative relative to the initial result value.

**[Table 2]**

| No. | Buttering Agent | pH | Sugar alcohol or Saccharide (w/v) | Surfactant (w/v) | Other |
|---|---|---|---|---|---|
| 1 | 20 mM sodium citrate | 4.8 | 5% mannitol | 0.02% polysorbate 20 | 0.1 mg/mL methionine |
| 2 | 20 mM sodium citrate | 5.1 | 5% mannitol | 0.02% polysorbate 20 | 0.1 mg/mL methionine |
| 3 | 20 mM sodium citrate | 5.4 | 5% mannitol | 0.02% polysorbate 20 | 0.1 mg/mL methionine |
| 4 | 20 mM sodium citrate | 5.7 | 5% mannitol | 0.02% polysorbate 20 | 0.1 mg/mL methionine |
| 5 | 20 mM sodium citrate | 6.0 | 5% mannitol | 0.02% polysorbate 20 | 0.1 mg/mL methionine |
| 6 | 20 mM sodium acetate | 4.8 | 5% mannitol | 0.02% polysorbate 20 | 0.1 mg/mL methionine |
| 7 | 20 mM sodium acetate | 5.1 | 5% mannitol | 0.02% polysorbate 20 | 0.1 mg/mL methionine |
| 8 | 20 mM sodium acetate | 5.4 | 5% mannitol | 0.02% polysorbate 20 | 0.1 mg/mL methionine |
| 9 | 20 mM sodium acetate | 5.7 | 5% mannitol | 0.02% polysorbate 20 | 0.1 mg/mL methionine |
| 10 | 20 mM sodium acetate | 6.0 | 5% mannitol | 0.02% polysorbate 20 | 0.1 mg/mL methionine |

**[Table 3]**

| No. | IE-HPLC (%) | | | RP-HPLC (%) | | | SE-HPLC (%) | | |
|---|---|---|---|---|---|---|---|---|---|
| | Initial | 2 weeks | 5 weeks | Initial | 2 weeks | 5 weeks | Initial | 2 weeks | 5 weeks |
| 1 | 100 | 89.7 | 71.1 | 100 | 98.0 | 92.6 | 100 | 99.8 | 99.4 |
| 2 | 100 | 92.4 | 76.0 | 100 | 99.2 | 94.9 | 100 | 99.7 | 99.4 |
| 3 | 100 | 93.8 | 74.8 | 100 | 98.1 | 94.6 | 100 | 99.7 | 99.2 |
| 4 | 100 | 90.2 | 66.5 | 100 | 98.0 | 95.0 | 100 | 99.5 | 98.6 |
| 5 | 100 | 84.2 | 54.1 | 100 | 97.8 | 94.3 | 100 | 99.2 | 98.2 |
| 6 | 100 | 82.9 | 64.8 | 100 | 96.9 | 96.8 | 100 | 99.7 | 99.1 |
| 7 | 100 | 85.9 | 70.1 | 100 | 97.0 | 96.7 | 100 | 99.8 | 99.4 |
| 8 | 100 | 88.5 | 74.4 | 100 | 96.8 | 96.4 | 100 | 99.8 | 99.5 |
| 9 | 100 | 91.5 | 78.1 | 100 | 97.3 | 97.1 | 100 | 99.8 | 99.5 |
| 10 | 100 | 92.8 | 79.5 | 100 | 97.5 | 97.5 | 100 | 99.8 | 99.5 |

As can be seen from the above results, it was confirmed that there was a change in stability according to pH in the presence of sodium citrate and sodium acetate buffer solutions.

### Example 5: Evaluation of Stability of Long-Acting Conjugates of GLP-2 Derivative According to Types of Stabilizers and pH

Based on the composition (sodium acetate, polysorbate 20, and methionine) of the above liquid formulation, the stability of the long-acting conjugate of GLP-2 derivative was compared according to the types of stabilizers and pH.

For the stabilizer, sorbitol and sucrose were added in addition to mannitol confirmed in Example 1, and the stability of the long-acting conjugate of the GLP-2 derivative was compared by comparing pH of 5.1 and 6.0.

In particular, the concentrations of mannitol, sorbitol and sucrose were selected in consideration of the maximum allowable range of commercially available formulations and that recommended by the licensing agency.

A liquid formulation of the long-acting conjugate of GLP-2 derivative (CA-GLP-2 RK(3.4K PEG) was prepared using the composition shown in Table 4 (the concentration of the long-acting conjugate in the liquid formulation is about 1,117.8 nmol/mL) and stored at 25°C for 4 weeks, and then was analyzed by IE-HPLC, RP-HPLC, and SE-HPLC.

In Table 5, IE-HPLC (%), RP-HPLC (%), and SE-HPLC (%) indicate Area% / Start Area% (%), indicating the residual ratio of the long-acting conjugate of GLP-2 derivative relative to the initial result value.

**[Table 4]**

| No. | Buttering Agent | pH | Sugar alcohol or Saccharide (w/v) | Surfactant (w/v) | Other |
|---|---|---|---|---|---|
| 1 | 20 mM sodium acetate | 6.0 | 5% mannitol | 0.02% polysorbate 20 | 0.1 mg/mL methionine |
| 2 | 20 mM sodium acetate | 6.0 | 5% sorbitol | 0.02% polysorbate 20 | 0.1 mg/mL methionine |
| 3 | 20 mM sodium acetate | 6.0 | 8% sucrose | 0.02% polysorbate 20 | 0.1 mg/mL methionine |
| 4 | 20 mM sodium acetate | 5.1 | 5% mannitol | 0.02% polysorbate 20 | 0.1 mg/mL methionine |
| 5 | 20 mM sodium acetate | 5.1 | 5% sorbitol | 0.02% polysorbate 20 | 0.1 mg/mL methionine |
| 6 | 20 mM sodium acetate | 5.1 | 8% sucrose | 0.02% polysorbate 20 | 0.1 mg/mL methionine |

**[Table 5]**

| No. | IE-HPLC (%) | | | RP-HPLC (%) | | | SE-HPLC (%) | | |
|---|---|---|---|---|---|---|---|---|---|
| | Initial | 2 weeks | 4 weeks | Initial | 2 weeks | 4 weeks | Initial | 2 weeks | 4 weeks |
| 1 | 100 | 89.1 | 83.9 | 100 | 96.3 | 95.6 | 100 | 99.8 | 99.8 |
| 2 | 100 | 89.5 | 85.3 | 100 | 96.4 | 95.6 | 100 | 99.7 | 99.7 |
| 3 | 100 | 89.6 | 85.4 | 100 | 96.7 | 95.8 | 100 | 99.7 | 99.9 |
| 4 | 100 | 84.1 | 77.9 | 100 | 96.5 | 95.3 | 100 | 100.0 | 99.9 |
| 5 | 100 | 84.0 | 76.8 | 100 | 96.7 | 94.6 | 100 | 99.8 | 99.7 |
| 6 | 100 | 82.8 | 74.3 | 100 | 96.6 | 94.9 | 100 | 100.0 | 100.0 |

As can be seen from the above results, the long-acting conjugate of the GLP-2 derivative was stable in the liquid formulation of the present invention.

### Example 6: Long-Term Storage Stability Test for High-Concentration Liquid Formulation of Long-Acting Conjugate of GLP-2 Derivative Using Final Formulation Composition

Long-term storage stability of high-concentration liquid formulations for three concentrations of the long-acting conjugate of GLP-2 derivative (CA-GLP-2 RK(3.4K PEG)) was analyzed using a liquid formulation containing sodium acetate of pH 6.0, sucrose, polysorbate 20, and methionine, which was ultimately selected through the previous Examples. Table 6 shows the results of long-term storage stability of high-concentration liquid formulations stored at 5°C ± 3°C for 12 months, and RP-HPLC (%) and SE-HPLC (%) indicate Area% / Start Area% (%), indicating the residual ratio of the long-acting conjugate of GLP-2 derivative relative to the initial result value.

**[Table 6]**

| | | | | | | |
|---|---|---|---|---|---|---|
| Formulation Composition | 20 mM sodium acetate (pH 6.0), 8% sucrose, 0.02% polysorbate 20, 0.1 mg/mL methionine | | | | | |
| Storage Temperature | 5°C ± 3°C | | | | | |
| Formulation Concentration | 558.9 nmol/mL | 1,490.4 nmol/mL | 2,980.8 nmol/mL | | | |

| | RP-HPLC (%) | SE-HPLC (%) | RP-HPLC (%) | SE-HPLC (%) | RP-HPLC (%) | SE-HPLC (%) |
|---|---|---|---|---|---|---|
| 0 month | 100 | 100 | 100 | 100 | 100 | 100 |
| 1 month | 99.3 | 100.2 | 100.2 | 100.2 | 101.1 | 100.1 |
| 3 months | 99.8 | 100.4 | 100.5 | 100.1 | 101.4 | 100.1 |
| 6 months | 98.2 | 100.5 | 98.5 | 99.7 | 98.7 | 99.6 |
| 9 months | 97.5 | 100.1 | 97.8 | 99.9 | 99.1 | 99.7 |

As a result of the long-term storage stability test, it was confirmed that the long-acting conjugate of GLP-2 derivative at three high concentrations was stable for more than 9 months in the liquid formulation of the present invention.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A liquid formulation of a long-acting conjugate of GLP-2, comprising:
18 nmol/mL to 18,630 nmol/mL of a long-acting conjugate of GLP-2 represented by Chemical Formula 1 below, and
a stabilizer comprising 1% (w/v) to 20% (w/v) of a sugar alcohol, a saccharide, or a combination thereof; and a buffering agent in an amount for maintaining the pH in the range of 4.5 to 6.5:
[Chemical Formula 1] X-Lₐ-F
wherein,
X is native GLP-2 or a GLP-2 derivative;
L is a linker containing an ethylene glycol repeating unit;
a is 0 or a natural number, with the proviso that when a is 2 or more, each L is independent of each other;
F is an immunoglobulin Fc fragment; and
- represents a covalent bond.

2. The liquid formulation of claim 1, wherein the GLP-2 derivative has a variation selected from the group consisting of substitution, addition, deletion, modification, and a combination thereof in one or more amino acids in the native GLP-2 sequence.

3. The liquid formulation of claim 1, wherein the X comprises an amino acid sequence represented by General Formula 1 below:
[General Formula 1] X₁X₂DGSFSDEMNTILDNLAARDFINWLIQTX₃₀ITDX₃₄ (SEQ ID NO: 9)
wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent, or lysine, arginine, glutamine, histidine, 6-azidolysine or cysteine.

4. The liquid formulation of claim 3, wherein the X is:
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine.

5. The liquid formulation of claim 1, wherein the X comprises an amino acid sequence represented by General Formula 2 below:
[General Formula 2] X₁X₂DGSFSDEMNTILDNLAARDFINWLIQTX₃₀ITDX₃₄ (SEQ ID NO: 10)
wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is any one or more amino acids or any one or more amino acids in which a variation has occurred.

6. The liquid formulation of claim 1, wherein the GLP-2 derivative comprises an amino acid sequence selected from the group consisting of SEQ ID NOS: 2 to 8.

7. The liquid formulation of claim 1, wherein the immunoglobulin Fc fragment is an aglycosylated IgG4 Fc region.

8. The liquid formulation of claim 1, wherein the F is a dimer consisting of two polypeptide chains, and one end of the L is linked to only one of the two polypeptide chains.

9. The liquid formulation of claim 1, wherein the L is polyethylene glycol.

10. The liquid formulation of claim 1, wherein the formula weight of the ethylene glycol repeating unit moiety in the L is in the range of 1 kDa to 100 kDa.

11. The liquid formulation of claim 1, wherein the immunoglobulin Fc fragment comprises the amino acid sequence of SEQ ID NO: 32.

12. The liquid formulation of claim 1, wherein the pH of the liquid formulation is in the range of 4.8 to 6.0.

13. The liquid formulation of claim 1, wherein the stabilizer further comprises one or more components selected from the group consisting of a nonionic surfactant and an amino acid.

14. The liquid formulation of claim 1, wherein the buffering agent is selected from the group consisting of citric acid and a salt thereof, acetic acid and a salt thereof, histidine and a salt thereof, phosphoric acid and a salt thereof, and a combination thereof.

15. The liquid formulation of claim 1, wherein the concentration of the buffering agent is 5 mM to 100 mM.

16. The liquid formulation of claim 1, wherein the sugar alcohol is one or more selected from the group consisting of mannitol and sorbitol.

17. The liquid formulation of claim 1, wherein the saccharide is glucose, fructose, galactose, lactose, maltose, sucrose, or a combination thereof.

18. The liquid formulation of claim 13, wherein the nonionic surfactant is poloxamer, polysorbate, or a combination thereof.

19. The liquid formulation of claim 13, wherein the nonionic surfactant is selected from the group consisting of poloxamer 188, polysorbate 20, polysorbate 40, polysorbate 60, polysorbate 80, and a combination thereof.

20. The liquid formulation of claim 13, wherein the nonionic surfactant is present in a concentration of 0.001% (w/v) to 0.2% (w/v) in the liquid formulation.

21. The liquid formulation of claim 13, wherein the amino acid is selected from the group consisting of methionine, arginine, histidine, glycine, cysteine, and a combination thereof.

22. The liquid formulation of claim 13, wherein the amino acid is present in a concentration of 0.01 mg/mL to 1 mg/mL in the liquid formulation.

23. The liquid formulation of claim 1, wherein the liquid formulation comprises:
18 nmol/mL to 18,630 nmol/mL of a long-acting conjugate of GLP-2;
5 mM to 100 mM of a buffering agent;
1% (w/v) to 20% (w/v) of a sugar alcohol, a saccharide, or a combination thereof;
0.001% (w/v) to 0.2% (w/v) of a nonionic surfactant; and
0.01 mg/mL to 1 mg/mL of an amino acid, and
wherein the pH of the liquid formulation is 4.5 to 6.5.

24. The liquid formulation of claim 1, wherein the liquid formulation comprises:
18 nmol/mL to 18,630 nmol/mL of a long-acting conjugate of GLP-2;
5 mM to 100 mM of a buffering agent selected from the group consisting of citric acid and a salt thereof, acetic acid and a salt thereof, histidine and a salt thereof, phosphoric acid and a salt thereof, and a combination thereof;
1% (w/v) to 20% (w/v) of glucose, fructose, galactose, lactose, maltose, sucrose, mannitol, sorbitol, or a combination thereof;
0.001% (w/v) to 0.2% (w/v) of a nonionic surfactant selected from the group consisting of poloxamer, polysorbate, and a combination thereof; and
0.01 mg/mL to 1 mg/mL of an amino acid selected from the group consisting of methionine, arginine, histidine, glycine, cysteine, and a combination thereof, and
wherein the pH of the liquid formulation is 4.5 to 6.5.

25. The liquid formulation of claim 1, wherein the liquid formulation comprises:
186 nmol/mL to 2,981 nmol/mL of a long-acting conjugate of GLP-2;
10 mM to 40 mM of a buffering agent of acetic acid and a salt thereof, or citric acid and a salt thereof;
1% (w/v) to 20% (w/v) of sucrose, mannitol, sorbitol, or a combination thereof;
0.002% (w/v) to 0.05% (w/v) of polysorbate; and
0.02 mg/mL to 0.5 mg/mL of methionine, and
wherein the pH of the liquid formulation is 4.8 to 6.0.

26. The liquid formulation of claim 1, further comprising polyhydric alcohol.

27. A method for preparing the liquid formulation of any one of claims 1 to 26, comprising: mixing (i) the long-acting conjugate of GLP-2, in which GLP-2 and the immunoglobulin Fc fragment are linked to each other; with (ii) a stabilizer comprising a sugar alcohol, a saccharide, or a combination thereof; and a buffering agent.
